# FASCICULE DE BREVET EUROPEEN

(11) **EP 1 718 730 B1**
(45) Date de publication et mention de la délivrance du brevet: **23.12.2009**
(21) Numéro de dépôt: 05732472.5
(22) Date de dépôt: 28.02.2005
(51) Int. Cl.: C12N 1/02, C12M 1/12, A23L 1/30

(54) **CONCENTRAT LIQUIDE DE BACTERIES ADAPTEES ET VIABLES POUR UN USAGE ALIMENTAIRE**
FLÜSSIGKONZENTRAT VON BAKTERIEN, DIE FÜR DEN VERZEHR ADAPTIERT UND GEEIGNET SIND
LIQUID CONCENTRATE OF BACTERIA THAT ARE ADAPTED AND FIT FOR ALIMENTARY USE

(30) Priorité: 27.02.2004 FR 0401997
(43) Date de publication de la demande: 08.11.2006
(60) Demande divisionnaire: 09176125.4
(73) Titulaire: Compagnie Gervais Danone, 75009 Paris (FR)
(72) Inventeur: TERRAGNO, Luc, F-75018 Paris (FR); CATONNET, Guillaume, F-91300 Massy (FR); REGULIER, Pascal, F-78280 Guyancourt (FR); DAVAL, Christophe, F-94600 Choisy le Roi (FR); TEISSIER, Philippe, F-91120 Palaiseau (FR); BARBEAU, Jean-Yves, F-91430 Igny (FR)
(74) Mandataire: Texier, Christian
(86) Numéro de dépôt international: PCT/FR2005/000478
(87) Numéro de publication internationale: WO 2005/090551

(56) Documents cités:
- SHAH N P: "Probiotic bacteria: selective enumeration and survival in dairy foods." JOURNAL OF DAIRY SCIENCE. APR 2000, vol. 83, no. 4, avril 2000 (2000-04), pages 894-907, XP002299123 ISSN: 0022-0302
- MARGOLLES ABELARDO ET AL: "Characterisation of a Bifidobacterium strain with acquired resistance to cholate--a preliminary study." INTERNATIONAL JOURNAL OF FOOD MICROBIOLOGY. 25 APR 2003, vol. 82, no. 2, 25 avril 2003 (2003-04-25), pages 191-198, XP002299124 ISSN: 0168-1605
- KIM W S ET AL: "Assessment of stress response of the probiotic Lactobacillus acidophilus." CURRENT MICROBIOLOGY. NOV 2001, vol. 43, no. 5, novembre 2001 (2001-11), pages 346-350, XP002299125 ISSN: 0343-8651
- CRESPO J P S G ET AL: "TANGENTIAL FLOW FILTRATION FOR CONTINUOUS CELL RECYCLE CULTURE OF ACIDOGENIC BACTERIA" CHEMICAL ENGINEERING SCIENCE, OXFORD, GB, vol. 47, no. 1, 1992, pages 205-214, XP009034901 ISSN: 0009-2509
- CARIDIS K A ET AL: "Pressure effects in cross-flow microfiltration of suspensions of whole bacterial cells" BIOPROCESS ENGINEERING, vol. 16, no. 4, 1997, pages 199-208, XP002299126 ISSN: 0178-515X
- TANNY G B ET AL: "IMPROVED FILTRATION TECHNIQUE FOR CONCENTRATING AND HARVESTING BACTERIA" APPLIED AND ENVIRONMENTAL MICROBIOLOGY, vol. 40, no. 2, 1980, pages 269-273, XP009037575 ISSN: 0099-2240
- REID D E ET AL: "LARGE-SCALE HARVESTING AND CONCENTRATION OF BACTERIA BY TANGENTIAL FLOW FILTRATION" JOURNAL OF APPLIED BACTERIOLOGY, vol. 41, no. 2, 1976, pages 321-324, XP009037592 ISSN: 0021-8847

## Description

La présente invention concerne un concentrât liquide de bactéries adaptées et viables pour un usage alimentaire. Les bactéries produites sont des bactéries lactiques.

L'ingestion de certaines souches de bactéries, en particulier celles qui appartiennent aux genres *Lactobacillus* et *Bifidobacterium* sont particulièrement bénéfiques au niveau de la santé, notamment en favorisant un bon fonctionnement de la flore intestinale. En effet, ces bactéries produisent des bactériocines et de l'acide lactique qui augmentent la digestibilité des aliments, favorisent le péristaltisme intestinal, et accélèrent l'évacuation des selles. De plus, ces bactéries produisent certaines vitamines du complexe B, et favorisent en général l'absorption des vitamines et minéraux, diminuent le cholestérol sanguin, renforcent le système immunitaire et tapissent les muqueuses intestinales afin de protéger contre l'invasion et les activités des microorganismes nuisibles.

De ce fait, depuis plusieurs années, les industries agroalimentaires tentent d'incorporer de telles bactéries dans leurs produits finaux, le plus généralement des yaourts.

Actuellement, ces bactéries sont utilisées sous une forme congelée ou lyophilisée. Cependant, ces processus de production sont traumatisants pour les bactéries qui perdent une partie de leur activité et parfois leur viabilité. Cela est préjudiciable pour les industriels producteurs et pour les consommateurs de ces produits car les bactéries doivent satisfaire aux exigences de qualité et de performances technologiques, si possible pendant plusieurs mois. Il serait donc souhaitable d'utiliser des bactéries produites par un procédé leur assurant une viabilité et une activité maximale. A cet effet, une méthode consiste à produire les bactéries sous une forme liquide. Cependant il a été mis en évidence que cette méthode génère également une mortalité importante des bactéries, après l'introduction des bactéries dans le produit final.

En outre, pour diminuer les coûts de stockage des bactéries et faciliter l'adjonction des bactéries dans le produit final, il serait souhaitable de concentrer les bactéries sous forme liquide. Pour cela, l'homme du métier utilise de manière habituelle une étape de centrifugation ou de filtration. Cependant, la centrifugation est un processus traumatisant pour les bactéries qui peut entraîner une forte mortalité cellulaire notamment due à un fort cisaillement et de plus, ce procédé n'est pas bien adapté pour la centrifugation de faibles volumes tels que ceux requis dans la production de bactéries destinées à être additionnées en tant que probiotique à des produits alimentaires. Concernant une étape de filtration classique, celle-ci pose également des problèmes mortalité des bactéries et de colmatage des filtres par les bactéries.

Il serait donc souhaitable de produire un volume souhaité de concentrât liquide de bactéries qui présentent une activité et une viabilité maximale après l'étape de concentration et après l'introduction dans le produit final.

Les inventeurs ont montré qu'une étape d'adaptation des bactéries permettait d'augmenter de manière significative l'activité et la viabilité des bactéries après l'introduction dans le produit final.

De plus, les inventeurs ont montré qu'une étape de filtration tangentielle, sous certaines conditions particulières (pression, concentration, porosité de membrane etc), permettait de concentrer de gros volumes de culture de bactéries, tout en préservant leur viabilité et sans colmatage des filtres.

La filtration tangentielle permet de produire deux courants en fonction de la nature et de la structure de la membrane : le perméat (le milieu de culture sensiblement exempt de bactéries) et le retentât (contenant les bactéries, appelé aussi concentrât). Dans une filtration tangentielle, le fluide ne circule non pas perpendiculairement mais parallèlement à la surface de la membrane et assure ainsi par sa vitesse d'écoulement un auto nettoyage qui prévient l'accumulation de dépôts qui obturent la surface de filtration (appelé communément colmatage des filtres).

Dans Shah (Journal of dairy Science, 2000, 83 :894-907), diverses approches pour améliorer la viabilité des probiotiques dans les yaourts sont passées en revue, entre autres l'adaptation au stress.

Margolles et al. (International Journal of Food Microbiology, 2003, 82 :191-198) ont sélectionné des souches de *Bifidobacterium* rendues résistantes aux sels de bile et au cholate de sodium par adaptation.

Kim et al. (Current Microbiology, 2001, 43 : 346-350) ont étudié différents aspects de la réponse au stress chez *Lactobacillus acidophilus*.

Des systèmes de recyclage de cellules par filtration tangentielle ont été analysés par Crespo et al. (Chemical Engineering Science, 1992, 47 :205-214), en s'intéressant plus particulièrement à des bactéries acidogéniques.

Caridis et al. (Bioprocess Engineering, 1997, 16: 199-208) ont évalué un système de microfiltration tangentielle à l'aide de membranes tubulaires en céramique, sur des suspensions de *Mycobacterium*.

Tanny et al. (Applied and Environmental Microbiology, 1980, 40 : 269-273) ont évalué une cartouche plissée de filtration tangentielle pour concentrer et collecter des bactéries pathogènes ou non appartenant aux espèces *Escherichia coli* et *Salmonella typhimurium*.

Reid et al. (Journal of Applied Bacteriology, 1976, 41 : 321 - 324) décrivent l'utilisation d'un système de filtration par flux tangentiel pour récupérer, concentrer et laver des suspensions de *Corynebacterium parvum* dans des conditions aseptiques.

Un objet de la présente invention est donc un concentrât de bactéries lactiques **caractérisé en ce que** le concentrât est liquide, en ce que les bactéries sont adaptées, viables et à une concentration comprise entre 5.10¹⁰ et 5.10¹¹ ufc/ml, et en ce qu'il est susceptible d'être obtenu par un procédé comprenant les étapes successives de propagation des bactéries dans un milieu de culture, d'adaptation des bactéries, de lavage du milieu de culture contenant les bactéries adaptées par microfiltration tangentielle, et de concentration en bactéries du milieu lavé par microfiltration tangentielle.

Par bactéries lactiques, on entend désigner préférentiellement selon la présente invention des bactéries du genre *Lactobacillus spp., Bifidobacterium spp., Streptococcus spp, Lactococcus spp*. et en particulier *Lactobacillus casei, Lactobacillus plantarum, Lactobacillus bulgaricus, Lactobacillus helveticus, Lactobacillus acidophilus, Bifidobacterium animalis, Bifidobacterium breve, Streptococcus thermophilus, Lactococcus lactis*.

Par bactéries adaptées, on entend désigner, selon la présente invention, des bactéries plus résistantes à différents stress, en particulier liés à différents stress physicochimiques.

Par bactéries adaptées et viables, on entend désigner, selon la présente invention, des bactéries dont le taux de survie après 28 jours dans un produit alimentaire, en particulier un produit laitier ou une boisson, est supérieur à 60% et avantageusement supérieur à 80%.

La viabilité des bactéries est mesurée par des techniques de numération connue de l'homme du métier comme par exemple la numération en masse, la numération en surface, les cellules de Malassez, le comptage direct, la turbidité, la néphélométrie, le comptage électronique, la cytométrie en flux, la fluorescence, l'impédimétrie, l'analyse d'images.

Selon la présente invention, le concentrât est **caractérisé en ce que** les bactéries adaptées présentent au moins l'une des caractéristiques suivantes quand elles sont ajoutées à un produit alimentaire :
i) un taux de survie supérieur à 80% après 14 jours dans un produit alimentaire à une température comprise entre 4°C et 45°C, ledit produit alimentaire ayant un pH compris entre 3 et 7 ou
ii) un taux de survie supérieur à 60% et avantageusement supérieur à 80% après 28 jours dans un produit alimentaire à une température comprise entre 4°C et 45°C, ledit produit alimentaire ayant un pH compris entre 3 et 7.

Selon la présente invention, le concentrât est **caractérisé en ce que** les bactéries présentent les deux caractéristiques i) et ii).

Selon la présente invention, le concentrât est caractérisé en ce que le produit alimentaire est un produit laitier et/ou une boisson.

Selon la présente invention, les bactéries du concentrât sont viables pendant une période comprise entre 4 et 6 semaines.

Selon la présente invention, le milieu de culture de l'étape de propagation est un milieu synthétique.

Par milieu synthétique, on entend désigner selon la présente invention un milieu dans lequel sont introduits des composants soumis à un contrôle quantitatif et qualitatif rigoureux.

Selon la présente invention, la solution utilisée à l'étape de lavage est adaptée à l'utilisation alimentaire du concentrât de bactéries et présente une pression climatique compatible avec la viabilité des bactéries.

Les inventeurs ont montré que l'étape d'adaptation des bactéries permet de réduire la mortalité des celles-ci, provoquée par le changement de milieu des bactéries entre leur milieu de culture et le produit alimentaire final à additiver.

Selon la présente invention l'adaptation des bactéries est mise en évidence par la mesure de paramètres du milieu de culture des bactéries et/ou de paramètres des bactéries. Selon la présente invention, les paramètres du milieu de culture sont préférentiellement le pH, la pression osmotique et/ou la température.

D'autres paramètres du milieu de culture des bactéries pour la mise en évidence de l'adaptation des bactéries sont possibles, comme par exemple la concentration en sucre du milieu bactérien.

Préférentiellement, dans le cas où le paramètre du milieu de culture est le pH, l'étape d'adaptation est réalisée par diminution du pH par acidification naturelle.

Afin d'effectuer l'étape d'adaptation des bactéries au pH par acidification naturelle, on peut par exemple mesurer la concentration en sucre du milieu de fermentation et au-delà d'une concentration seuil pour chaque espèce de bactéries, on sait que le pH n'est plus régulé et l'adaptation au milieu devient très aisée.

Ainsi, par exemple, si la concentration en sucre du milieu de fermentation de *Lactobacillus casei* est de 9 g/L, le pH n'est plus régulé et est environ égal à 5. Il devient alors plus aisé pour la souche adaptée d'être ajoutée à un nouveau milieu et ceci permet une viabilité plus importante des bactéries dans le milieu final.

En outre, selon la présente invention, la filtration tangentielle peut être utilisée pour l'étape d'adaptation des bactéries.

Selon la présente invention la ou les membranes de filtration tangentielle sont d'une porosité comprise entre 0.01 et 0,5µm et de manière préférentielle, entre 0.1 et 0.3 µm.

Ces membranes sont utilisées pour les étapes de lavage et de concentration du procédé et éventuellement l'étape d'adaptation des bactéries.

Les membranes de filtration sont caractérisées par:
- la porosité et l'épaisseur de la couche filtrante dont dépend le débit de perméat.
- le diamètre des pores et leur répartition dont dépend l'efficacité de séparation.
- le matériau employé dont dépend la résistance mécanique, chimique, thermique et la facilité, de nettoyage.

Par membrane de filtration, on entend désigner des membranes organiques ou minérales.

Les membranes organiques peuvent être composées entre autre d'acétate de cellulose, de polyamides aromatiques, de polysulfone, d'esters de cellulose, de cellulose, de nitrate de cellulose, de PVC, ou de Polypropylène.

Les membranes minérales peuvent être composées entre autre de céramique frittée, de métal fritté, de carbone, ou de verre.

Selon la présente invention, le paramètre des bactéries est la taille des bactéries.

Préférentiellement, dans le cas où l'adaptation est mise en évidence par la taille des bactéries, la distribution des longueurs de chaque bactérie dudit concentrât se situe majoritairement entre 0,1 et 10 micromètres, avantageusement entre 0,5 et 5 micromètres.
La mesure de la taille des bactéries se fait par un moyen adapté.
Un moyen adapté peut être par exemple un prélèvement régulier de bactéries suivi d'une mesure de la taille des bactéries par cytométrie de flux.

Selon la présente invention, le pH du concentrât est compris entre 3 et 6.

Selon la présente invention, la température d'utilisation du concentrât est comprise entre 25 et 45°C et préférentiellement entre 35 et 39°C.

Par température d'utilisation, on entend désigner selon la présente invention la température du concentrât quand il est ajouté à un produit alimentaire.

Selon la présente invention, le concentrât est conditionné dans des poches souples, hermétiques et stériles.

Par poches souples et hermétiques, on entend désigner, selon la présente invention et de manière préférentielle, des poches en plastique alimentaire.

Selon la présente invention, le concentrât, conditionné en poches souples hermétiques, peut être conservé à une température comprise entre -50°C et 4°C après conditionnement.

De manière optionnelle, il est possible de rajouter au concentrât liquide de bactéries adaptées et viables conditionné en poches souples et hermétiques, et conservé à des températures basses, des molécules cryoprotectrices telles que le saccharose par exemple.

Selon la présente invention, le concentrât, conditionné en poches souples et hermétiques, conservé à une température comprise entre -50°C et 4°C, est réchauffé jusqu'à une température comprise entre 25°C et 45°C, avantageusement entre 35 et 39°C par un moyen adapté avant d'être utilisé.

Par moyen adapté, on entend désigner selon la présente invention par exemple l'utilisation d'un bain marie à une température non létale pour les bactéries, par exemple 37°C.

Un objet de la présente invention est également l'utilisation du concentrat liquide de bactéries adaptées et viables, selon la présente invention en tant qu'additif alimentaire.

Par additif alimentaire, on entend désigner selon la présente invention toute substance chimique ajoutée aux aliments durant leur préparation ou en vue de leur entreposage pour obtenir un effet technique désiré. De plus, selon la présente invention, le concentrât liquide de bactéries possède une numération stable, les bactéries étant viables et n'effectuant pas de fermentation dans le produit final additivé.

Un objet de la présente invention est également un produit alimentaire additivé, **caractérisé en ce que** l'additif alimentaire utilisé est le concentrât liquide de bactéries adaptées et viables selon la présente invention.

Selon la présente invention, le produit alimentaire est un produit laitier et/ou une boisson.

Par produit laitier on entend désigner selon la présente invention, en plus du lait, les produits dérivés du lait, tels la crème, la crème glacée, le beurre, le fromage, le yaourt; les produits secondaires, comme le lactosérum, la caséine ainsi que divers aliments préparés contenant comme ingrédient principal du lait ou des constituants du lait.

Par boisson on entend désigner selon la présente invention des boissons comme par exemple les jus de fruits, les mélanges de lait et de jus de fruits, les jus végétaux tels que par exemple le jus de soja, le jus d'avoine ou le jus de riz, les boissons alcoolisées comme par exemple le kéfir, les sodas, et les eaux de source ou minérales additionnées ou non de sucre ou d'arômes par exemple.

Un objet de la présente invention est également un procédé de fabrication d'un produit alimentaire additivé selon la présente invention, **caractérisé en ce que** le concentrât liquide de bactéries adaptées et viables est additionné au produit alimentaire en fin de ligne de production et préférentiellement avant le conditionnement du produit alimentaire.

Selon la présente invention, le procédé de fabrication d'un produit alimentaire additivé est **caractérisé en ce que** le concentrât liquide de bactéries adaptées et viables est additionné au produit alimentaire en ligne par pompage.

La présente invention sera mieux comprise à l'aide du complément de description qui va suivre, qui se réfère à des exemples de mesure de la viabilité et de l'adaptation des bactéries du concentrât liquide selon la présente invention.

Il va de soi, toutefois que ces exemples ne sont donnés qu'à titre d'illustration de l'objet de l'invention, dont ils ne sauraient constituer en aucune manière une limitation.

### Légende des figures :

- Figure 1: : Suivi de la viabilité de souches de lactobacillus casei adaptées et non adaptées dans un produit alimentaire de type yaourt sur une période de 28 jours.
- Figure 2a: : Histogramme de distribution de taille de souches de *Lactobacillus casei* adaptées et non adaptées avant d'être soumises à un stress acide.
- Figure 2b: : Histogramme de distribution de taille de souches de *Lactobacillus casei* adaptées et non adaptées après être soumises à un stress acide
- Figure 3: : Courbes montrant l'adaptation de *Lactobacillus casei* par rapport à la température du milieu de culture (37°C et 39°C). La permittivité relative ε _{R} (quantité adimensionnelle égale à la permittivité ε, exprimée en pF/cm, divisée par la permittivité du vide ε₀) est exprimée en fonction de l'age des bactéries (en heures)
- Figure 4: : Courbes montrant l'adaptation de *Lactobacillus casei* en fonction de la pression osmotique (concentration en glucose de 20, 40 et 80 g/L, respectivement en gris clair, gris foncé et noir). La permittivité ε, exprimée en pF/cm, est exprimée en fonction de la Densité Optique (DO).

### EXEMPLES :

### * Exemple 1 : Conséquences de l'adaptation de souches de L. casei sur leur viabilité.

On souhaite évaluer les conséquences d'une étape d'adaptation de souches de *Lactobacillus casei* au niveau de leur viabilité.

Pour cela, on prépare un lot de bactéries témoin de *Lactobacillus casei* qui sont mises en culture dans un milieu MRS (milieu spécial permettant la croissance des lactobacilles, mis au point par Man, de Rogosa et Sharpe).

En parallèle à ce lot témoin, on prépare un lot de bactéries *Lactobacillus casei*, qui après la mise en culture dans un milieu MRS sont adaptées par une étape d'acidification naturelle.

Pour cela, après 17 heures de culture, une diminution du pH est réalisée par acidification naturelle sur une heure pour passer de pH 6,5 à pH 5.

Ensuite, les deux lots de bactéries sont lavés et concentrés en bactéries par microfiltration tangentielle.

Ces deux concentrâts de bactéries sont introduits séparément dans une masse de yaourt à pH 5.5 et à une température de 10°C.

On mesure la quantité de bactéries vivantes à J+ 1 dans les deux lots de yaourt.

On prélève ensuite tous les jours un échantillon des deux lots de yaourt additionné respectivement du concentrât bactérien témoin et du concentrât de bactéries adaptées, et on quantifie le nombre de souches survivantes par rapport au nombre de souches vivantes à J+1. Pour cela, on effectue une numération en masse.

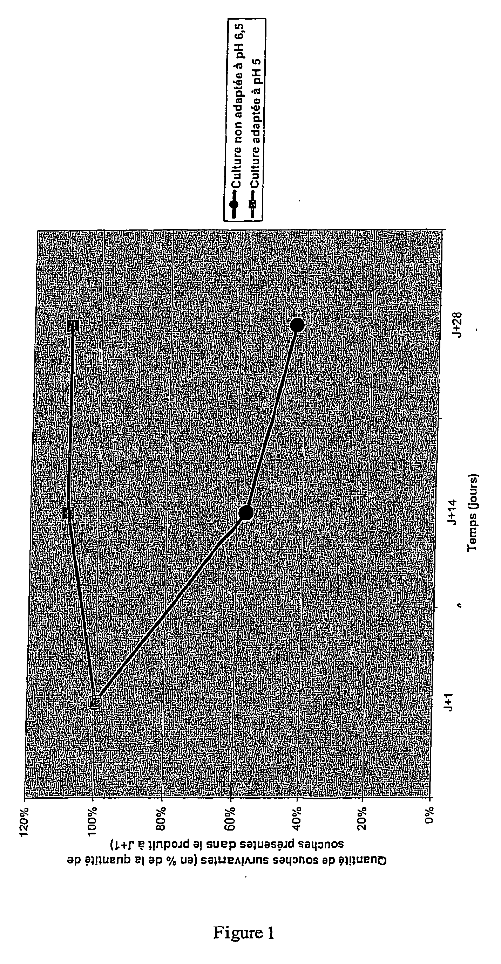

Pour chacune des mesures de viabilité durant la période de conservation du produit fini, ce dernier est bien homogénéisé avant le prélèvement. Un prélèvement stérile de 1 ml de produit est réalisé. Une dilution sériée de 10 en 10 est effectuée. Les différentes dilutions du produit sont placées dans une boite de Petri et un milieu gélosé liquide (puisque préalablement chauffé à 50°C) est coulé sur ces fractions du produit. On choisira le milieu à couler en fonction de la nature des bactéries que l'on souhaite compter. Le milieu gélosé durcit. Les boites de Petri sont alors placées en incubation pendant quelques jours (2-5j) à 37°C. Les résultats sont illustrés par la figure 1.

On observe qu'au bout de 7 jours, le nombre de bactéries survivantes dans le lot des bactéries témoin est de 80% et dans le lot de bactéries adaptées de 105% (il y a eu une légère croissance bactérienne).

Au bout de 14 jours, le nombre de bactéries survivantes dans le lot des bactéries témoin est de 58% et dans le lot de bactéries adaptées de 110% (il y a eu une légère croissance bactérienne).

Au bout de 28 jours, le nombre de bactéries survivantes dans le lot des bactéries témoin est de 42% et dans le lot de bactéries adaptées de 110% (il y a eu une légère croissance bactérienne).

En conclusion, l'étape d'adaptation des bactéries induit une diminution de la mortalité des bactéries de l'ordre de 60 % par rapport à un lot témoin de bactéries non adaptées, après 28 jours dans un yaourt.

### * Exemple 2 : Evolution de la taille de bactéries adaptées et de bactéries non adaptées soumises à un stress acide.

On souhaite suivre l'évolution de la taille de bactéries adaptées et de bactéries non adaptées soumises à un stress acide.

Pour cela, on prépare un lot de bactéries témoin de *Lactobacillus casei* qui sont mises en culture dans un milieu MRS (milieu spécial permettant la croissance des lactobacilles, mis au point par Man, de Rogosa et Sharpe).

En parallèle à ce lot témoin, on prépare un lot de bactéries *Lactobacillus casei,* qui après la mise en culture dans un milieu MRS sont adaptées par une étape d'acidification naturelle.

Pour cela, après 17 heures de culture, une descente du pH est réalisée par acidification naturelle sur une heure pour passer de pH 6,5 à pH 5.

Ensuite, les deux lots de bactéries sont lavés et concentrés en bactéries par microfiltration tangentielle.

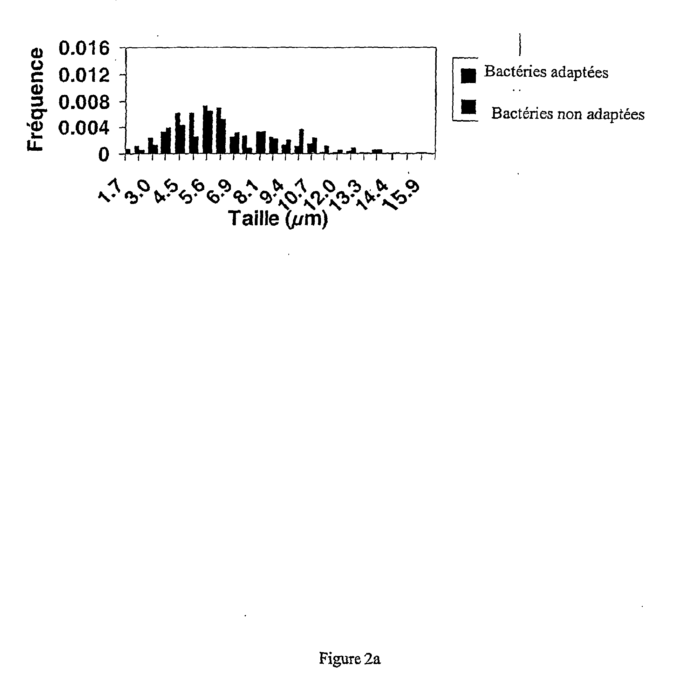

On effectue ensuite un prélèvement de bactéries et l'on mesure leur taille par cytométrie de flux. On établit ainsi un histogramme de distribution de taille des bactéries adaptées et non adaptées (lot témoin) (figure 2a). On observe que la distribution de taille des bactéries des deux lots est très similaire.

Ensuite, les deux lots de bactéries sont soumis à un stress acide par ajout des bactéries à un milieu ayant un pH de 3.

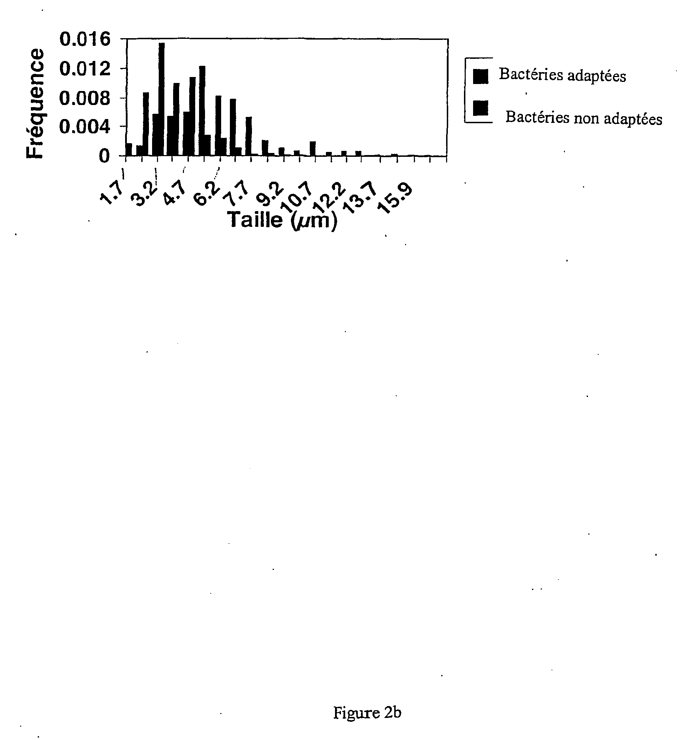

On effectue ensuite un prélèvement de bactéries et l'on mesure leur taille par cytométrie de flux. On établit ainsi un histogramme de distribution de taille des bactéries adaptées et non adaptées après un stress acide (figure 2b). On observe que la distribution de taille des bactéries des deux lots est très différente. Dans le lot de bactéries adaptées, la taille de fréquence la plus importante est de 3,2 µm (fréquence de 0.016). Dans le lot de bactéries non adaptées, la taille de fréquence la plus importante est de 5.45 µm (fréquence de 0.012).

En conclusion, l'étape d'adaptation des bactéries induit une diminution de la taille des bactéries de l'ordre de 60% lorsque celles-ci sont soumises à un stress acide, par rapport à des bactéries non adaptées. Il est donc possible de mettre en évidence l'adaptation des bactéries par la mesure de leur taille.

### * Exemple 3 : Mise en évidence de l'adaptation des bactéries par la mesure de l'influence du paramètre « température » du milieu de culture

On souhaite mettre en évidence l'adaptation des bactéries en mesurant l'influence du paramètre du milieu de culture qu'est la température.

Pour cela, on prépare deux lots de bactéries de *Lactobacillus casei* à partir d'un même inoculum. Ces deux lots sont mis en culture dans deux milieux MRS (milieu spécial permettant la croissance des Lactobacilles, mis au point par Man, de Rogosa et Sharpe).

Une sonde de biomasse permettant de mesurer la permittivité relative ε_{R} est utilisée. Les sondes utilisables à cette fin sont connues de l'homme du métier (voir notamment FR2835921). La permittivité relative ε_{R} est une quantité adimensionnelle égale à la permittivité ε (exprimée en pF/cm) divisée par la permittivité du vide ε₀ (ε₀ = 8,854187. 10⁻² pF/cm). L'évolution de la permittivité relative est mesurée au cours du temps. La permittivité relative sera fonction du nombre de cellules vivantes et de la taille de ces cellules.

Les deux milieux de culture rigoureusement identiques et comportant la même quantité de bactéries sont cultivés l'un à 37°C et l'autre à 39°C.

La quantité de souches augmente au cours du temps, ce qui est normal.

Les inventeurs ont pu vérifier que le nombre de cellules total n'était pas différent dans les deux milieux de culture. Une technique classique de mesure de densité optique (du type spectromètre d'absorbance) peut être utilisée à cette fin ou bien une sonde optique Wedgewood (système mesurant dans le proche infrarouge la densité optique de suspensions microbiennes). L'absorbance du milieu mesurée par un spectromètre sera fonction de la quantité de cellules totale dans le milieu. Des techniques de dénombrement sur boite peuvent aussi être utilisées.

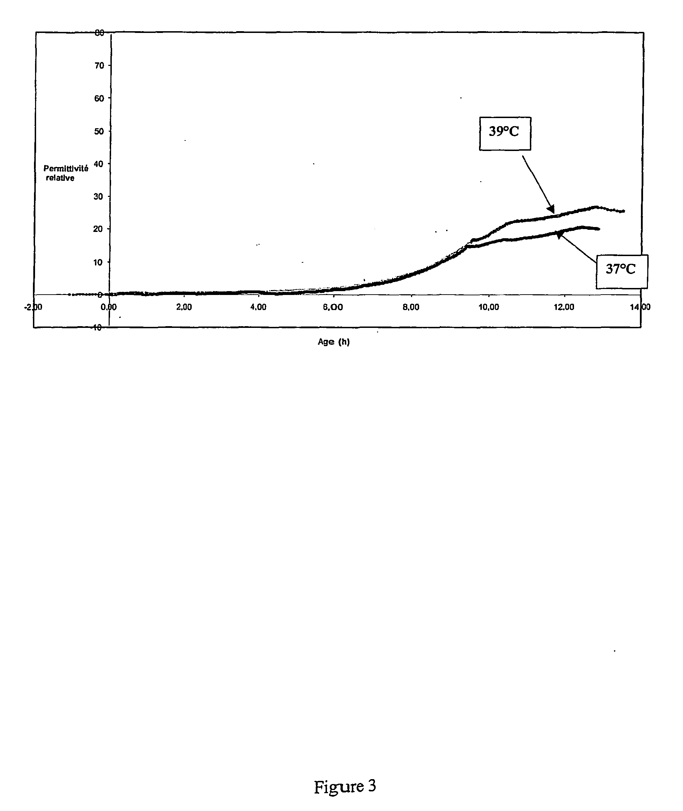

Grâce à la sonde de biomasse utilisée, il a pu être mis en évidence que les bactéries changeaient de forme et de taille, donc s'adaptaient en fonction de la température du milieu de culture. La Figure 3 illustre cette observation.

### * Exemple 4 : Mise en évidence de l'adaptation des bactéries par la mesure de l'influence des paramètres « température » et « pH » du milieu de culture.

On souhaite mettre en évidence l'adaptation des bactéries en mesurant l'influence de deux paramètres du milieu de culture pris conjointement que sont la température et le pH.

Pour cela, on prépare trois lots de bactéries de *Lactobacillus casei* à partir d'un même inoculum.

On cultivera les trois milieux de culture à trois températures différentes (35, 37 et 39°C) tout en soumettant à chaque fois les bactéries à un stress acide en abaissant le pH du milieu à un pH de 3.

Le changement de taille des cellules traduira leur adaptation aux conditions du milieu. Cette taille sera mesurée par microscopie.

Dans le Tableau 1, les résultats obtenus montrent bien que les bactéries s'adaptent en fonction des paramètres du milieu que sont la température et le pH puisque ces bactéries changent de taille.

**Tableau 1 : Influence de la température et du pH sur la taille des bactéries (exprimés en µm)**

| **Température (°C)** | **Taille moyenne des bactéries pendant la préculture** | **Taille moyenne des bactéries avant changement de pH** | **Taille moyenne des bactéries après changement de pH** |
|---|---|---|---|
| **35** | 3 | 3 | 2 |
| **37** | 4 | 5 | 4 |
| **39** | 3 | 7 | 5 |

### * Exemple 5 : Mise en évidence de l'adaptation des bactéries par la mesure de l'influence du paramètre « pression osmotique » du milieu de culture.

On souhaite mettre en évidence l'adaptation des bactéries en mesurant l'influence du paramètre du milieu de culture qu'est la pression osmotique.

Pour cela, on prépare trois lots de bactéries de *Lactobacillus casei* à partir d'un même inoculum. Ces lots sont mis en culture dans un milieu MRS (milieu spécial permettant la croissance des *Lactobacilles,* mis au point par Man, de Rogosa et Sharpe).

Les trois milieux de culture comportant la même quantité de bactéries contiendront respectivement des quantités de 20, 40 et 80 g de glucose par litre de milieu de culture. Plus la concentration en glucose du milieu est élevée, plus la pression osmotique de ce milieu est élevée.

Une sonde permettant de mesurer la permittivité relative est utilisée. Les sondes utilisables à cette fin sont connues de l'homme du métier (FR2835921). L'évolution de cette permittivité relative au cours du temps est mesurée. La permittivité ε (exprimée en pF/cm) est calculée en multipliant la permittivité relative ε_{R} mesurée par la permittivité du vide ε₀ (ε₀ = 8,854187. 10⁻² pF/cm).

Une technique classique de mesure de densité optique (du type spectromètre d'absorbance), ou bien une sonde optique Wedgewood (système mesurant dans le proche infra-rouge la densité optique de suspensions microbiennes), est utilisée pour mesurer l'évolution de la densité optique du milieu au cours du temps. La valeur de Densité Optique (DO) obtenue sera fonction du nombre de cellules total dans le milieu.

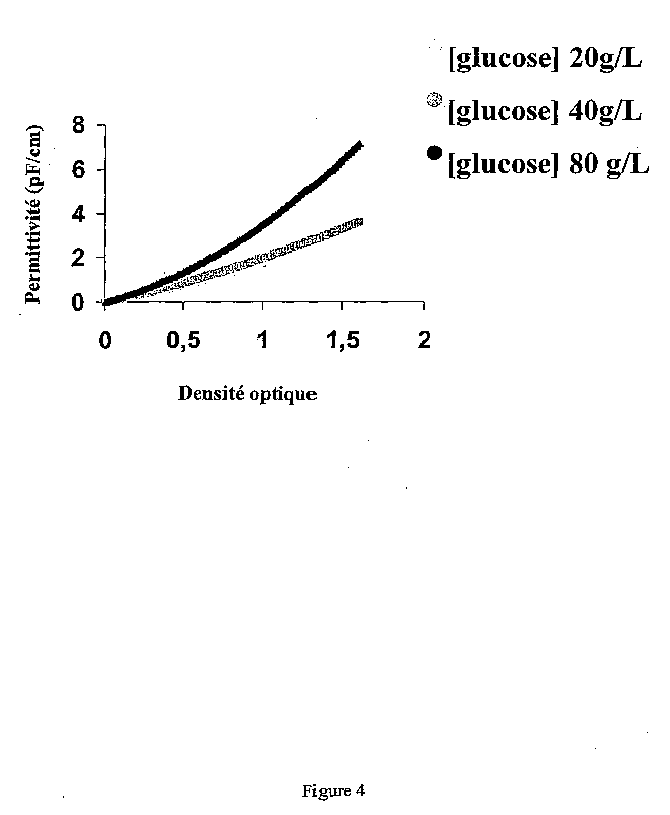

En exprimant la permittivité en fonction de la DO, la courbe résultante (Figure 4) permet de mettre en évidence l'évolution de la viabilité (exprimée par la mesure de la permittivité) et de la taille des cellules en fonction de la pression osmotique du milieu. Les résultats montrent que les bactéries changent de taille. En effet, dans le cas où il n'y aurait pas ce changement de taille des cellules, les résultats observés sur la Figure 4 seraient des droites rectilignes. Dans le cas présent, on peut observer des courbes (corrélation non linéaire).

Les bactéries s'adaptent donc en fonction de la pression osmotique du milieu de culture.

## Revendications

1. Concentrât liquide de bactéries lactiques, en particulier de bactéries du genre *Lactobacillus spp., Bifdobacterium spp., Streptococcus spp.* et *Lactococcus spp*., dans lequel les bactéries sont adaptées, viables et à une concentration entre 5.10¹⁰ et 5.10¹¹ ufc/ml, lesdites bactéries adaptées étant plus résistantes à différents stress, en particulier liés à différents stress physicochimiques, ledit concentrât étant susceptible d'être obtenu par un procédé comprenant les étapes successives de propagation des bactéries dans un milieu de culture, d'adaptation des bactéries, de lavage du milieu de culture contenant les bactéries adaptées par microfiltration tangentielle, et de concentration en bactéries du milieu lavé par microfiltration tangentielle.

2. Concentrât selon la revendication 1 **caractérisé en ce que** les bactéries adaptées présentent au moins l'une des caractéristiques suivantes quand elles sont ajoutées à un produit alimentaire :
i) un taux de survie supérieur à 80% après 14 jours dans un produit alimentaire à une température comprise entre 4°C et 45°C, ledit produit alimentaire ayant un pH compris entre 3 et 7 ou
ii) un taux de survie supérieur à 60% et avantageusement supérieur à 80% après 28 jours dans un produit alimentaire à une température comprise entre 4°C et 45°C, ledit produit alimentaire ayant un pH compris entre 3 et 7.

3. Concentrât selon la revendication 2 **caractérisé en ce que** les bactéries présentent les deux caractéristiques i) et ii).

4. Concentrât selon l'une quelconque des revendications 2 et 3 **caractérisé en ce que** le produit alimentaire est un produit laitier et/ou une boisson.

5. Concentrât selon l'une quelconque des revendications précédentes **caractérisé en ce que** les bactéries sont viables pendant une période comprise entre 4 et 6 semaines.

6. Concentrât selon l'une quelconque des revendications précédentes **caractérisé en ce que** l'adaptation des bactéries est mise en évidence par la mesure de paramètres du milieu de culture des bactéries et/ou de paramètres des bactéries.

7. Concentrât selon la revendication 6, **caractérisé en ce que** les paramètres du milieu de culture sont le pH, la pression osmotique, et/ou la température.

8. Concentrât selon la revendication 7 **caractérisé en ce que** le paramètre du milieu de culture est le pH et que l'étape d'adaptation est réalisée par diminution du pH par acidification naturelle.

9. Concentrât selon l'une quelconque des revendications 6 à 8, **caractérisé en ce que** le paramètre des bactéries est la taille des bactéries.

10. Concentrât selon la revendication 9 **caractérisé en ce que** la distribution des longueurs de chaque bactérie dudit concentrât se situe majoritairement entre 0,1 et 10 micromètres, avantageusement entre 0,5 et 5 micromètres.

11. Concentrât selon l'une quelconque des revendications précédentes **caractérisé en ce que** son pH est compris entre 3 et 6.

12. Concentrât selon l'une quelconque des revendications 1 à 11 **caractérisé en ce qu'**il est conservé à une température comprise entre -50°C à +4°C après conditionnement.

13. Concentrât selon la revendication 12 **caractérisé en ce qu'**il est réchauffé jusqu'à une température comprise entre 25 et 45°C, avantageusement entre 35 et 39°C par un moyen adapté avant d'être utilisé.

14. Utilisation du concentrât selon l'une quelconque des revendications 1 à 13 en tant qu'additif alimentaire.

15. Utilisation du concentrât selon l'une quelconque des revendications 1 à 13, à une température comprise entre 25 et 45°C, avantageusement entre 35 et 39°C.

16. Récipient sous la forme de poches souples, hermétiques et stériles comprenant le concentrât selon l'une quelconque des revendications 1 à 13.

17. Produit alimentaire additivé, **caractérisé en ce que** l'additif alimentaire utilisé est un concentrât liquide de bactéries adaptées et viables selon l'une quelconque des revendications 1 à 13.

18. Produit alimentaire additivé selon la revendication 17, **caractérisé en ce qu'**il s'agit d'un produit laitier et/ou d'une boisson.

19. Procédé de fabrication d'un produit alimentaire additivé selon l'une quelconque des revendications 17 et 18, **caractérisé en ce que** le concentrât liquide de bactéries adaptées et viables selon l'une quelconque des revendications 1 à 13 est additionné au produit alimentaire en fin de ligne de production et avantageusement avant le conditionnement du produit alimentaire.

20. Procédé de fabrication d'un produit alimentaire additivé selon la revendication 19, **caractérisé en ce que** le concentrât liquide de bactéries adaptées et viables est additionné au produit alimentaire en ligne par pompage.

## Claims

1. Liquid concentrate of lactic acid bacteria, in particular of bacteria of the *Lactobacillus spp*., *Bifidobacterium spp*., *Streptococcus spp*. and *Lactococcus spp*. genus, in which the bacteria are adapted, viable and at a concentration between 5.10¹⁰ and 5.10¹¹ ufc/ml, said adapted bacteria being more resistant to various stresses, in particular associated with various physicochemical stresses, wherein said concentrate is obtainable by a method including the successive steps of propagation of the bacteria in a culture medium, adaptation of the bacteria, washing of the culture medium containing the adapted bacteria by tangential microfiltration, and concentration of bacteria in the washed medium by tangential microfiltration.

2. Concentrate according to claim 1, **characterised in that** the adapted bacteria have at least one of the following characteristics when they are added to a food product:
i) a survival rate above 80% after 14 days in a food product at a temperature between 4°C and 45°C, with said food product having a pH between 3 and 7, or
ii) a survival rate above 60% and advantageously above 80% after 28 days in a food product at a temperature between 4°C and 45°C, with said food product having a pH between 3 and 7.

3. Concentrate according to claim 2, **characterised in that** the bacteria have both characteristics i) and ii).

4. Concentrate according to either one of claims 2 or 3, **characterised in that** the food product is a dairy product and/or a drink.

5. Concentrate according to any one of the previous claims, **characterised in that** the bacteria are viable for a period of between 4 and 6 weeks.

6. Concentrate according to any one of the previous claims, **characterised in that** the adaptation of the bacteria is determined by measuring parameters of the bacteria culture medium and/or parameters of the bacteria.

7. Concentrate according to claim 6, **characterised in that** the parameters of the culture medium are the pH, the osmotic pressure and/or the temperature.

8. Concentrate according to claim 7, **characterised in that** the parameter of the culture medium is the pH and the adaptation step is performed by reducing the pH by natural acidification.

9. Concentrate according to any one of claims 6 to 8, **characterised in that** the parameter of the bacteria is the size thereof.

10. Concentrate according to claim 9, **characterised in that** the distribution of lengths of each bacterium of said concentrate are primarily between 0.1 and 10 micrometers, and advantageously between 0.5 and 5 micrometers.

11. Concentrate according to any one of the previous claims, **characterised in that** its pH is between 3 and 6.

12. Concentrate according to any one of claims 1 to 11, **characterised in that** it is preserved at a temperature between -50°C and 4°C after packaging.

13. Concentrate according to claim 12, **characterised in that** it is reheated to a temperature between 25°C and 45°C, and advantageously between 35°C and 39°C, by appropriate means before being used.

14. Use of the concentrate according to any one of claims 1 to 13 as a food additive.

15. Use of the concentrate according to any one of claims 1 to 13 at a temperature between 25°C and 45°C, and advantageously between 35°C and 39°C.

16. Container in the form of a flexible, hermetically sealed and sterile bag containing the concentrate according to any one of claims 1 to 13.

17. Food product containing a food additive, **characterised in that** the food additive used is a liquid concentrate of adapted and viable bacteria according to any one of claims 1 to 13.

18. Food product according to claim 17, **characterised in that** it is a dairy product and/or a drink.

19. Method for producing a food product containing a food additive according to either one of claims 17 or 18, **characterised in that** the liquid concentrate of adapted and viable bacteria according to any one of claims 1 to 13 is added to the food product at the end of the production line and advantageously before packaging of the food product.

20. Method for producing a food product containing a food additive according to claim 19, **characterised in that** the liquid concentrate of adapted and viable bacteria is added to the food product in the line by pumping.

## Patentansprüche

1. Flüssiges Konzentrat aus Milchsäurebakterien, insbesondere aus Bakterien der Gattung Lactobacillus spp., Bifidobacterium spp., Streptococcus spp. und Lactococcus spp, in dem die Bakterien angepasst und lebensfähig sind und in einer Konzentration zwischen 5.10¹⁰ und 5.10¹¹ KBE/ml vorliegen, wobei die angepassten Bakterien widerstandsfähiger gegen verschiedene Stresssituationen sind, insbesondere in Verbindung mit verschiedenen physikochemischen Stresssituationen, wobei das Konzentrat durch ein Verfahren erhalten werden kann, umfassend die aufeinander folgenden Schritte des Vermehrens der Bakterien in einem Kulturmedium, des Anpassens der Bakterien, des Reinigens des Kulturmediums, das die angepassten Bakterien enthält, durch tangentiale Mikrofiltration und des Anreicherns des Mediums, das durch tangentiale Mikrofiltration gereinigt wurde, mit Bakterien.

2. Konzentrat nach Anspruch 1, **dadurch gekennzeichnet, dass** die angepassten Bakterien mindestens eine der folgenden Eigenschaften aufweisen, wenn sie einem Nahrungsmittel zugefügt werden:
i) eine Überlebensrate von mehr 80% nach 14 Tagen in einem Nahrungsmittel bei einer Temperatur zwischen 4 °C und 45 °C, wobei das Nahrungsmittel einen pH zwischen 3 und 7 aufweist, oder
ii) eine Überlebensrate von mehr 60% und vorteilhafterweise von mehr als 80% nach 28 Tagen in einem Nahrungsmittel bei einer Temperatur zwischen 4 °C und 45 °C, wobei das Nahrungsmittel einen pH zwischen 3 und 7 aufweist.

3. Konzentrat nach Anspruch 2, **dadurch gekennzeichnet, dass** die Bakterien die zwei Eigenschaften i) und ii) aufweisen.

4. Konzentrat nach einem der Ansprüche 2 und 3, **dadurch gekennzeichnet, dass** das Nahrungsmittel ein Molkereiprodukt und/oder ein Getränk ist.

5. Konzentrat nach einem der vorhergehenden Ansprüche, **dadurch gekennzeichnet, dass** die Bakterien während eines Zeitraums von 4 bis 6 Wochen lebensfähig sind.

6. Konzentrat nach einem der vorhergehenden Ansprüche, **dadurch gekennzeichnet, dass** die Anpassung der Bakterien durch die Messung der Parameter des Kulturmediums der Bakterien und/oder der Parameter der Bakterien nachgewiesen wird.

7. Konzentrat nach Anspruch 6, **dadurch gekennzeichnet, dass** die Parameter des Kulturmediums der pH, der osmotische Druck und/oder die Temperatur sind.

8. Konzentrat nach Anspruch 7, **dadurch gekennzeichnet, dass** der Parameter des Kulturmediums der pH ist und **dadurch**, dass der Schritt des Anpassens durch die Verringerung des pH durch natürliche Ansäuerung durchgeführt wird.

9. Konzentrat nach einem der Ansprüche 6 bis 8, **dadurch gekennzeichnet, dass** der Parameter der Bakterien die Größe der Bakterien ist.

10. Konzentrat nach Anspruch 9, **dadurch gekennzeichnet, dass** die Verteilung der Längen jeder Bakterie des Konzentrats zum größten Teil zwischen 0,1 und 10 Mikrometer, vorteilhafterweise zwischen 0,5 und 5 Mikrometer liegt.

11. Konzentrat nach einem der vorhergehenden Ansprüche, **dadurch gekennzeichnet, dass** sein pH zwischen 3 und 6 liegt.

12. Konzentrat nach einem der Ansprüche 1 bis 11, **dadurch gekennzeichnet, dass** es nach der Verpackung bei einer Temperatur zwischen -50 °C und +4 °C aufbewahrt wird.

13. Konzentrat nach Anspruch 12, **dadurch gekennzeichnet, dass** es bis zu einer Temperatur zwischen 25 und 45 °C, vorteilhafterweise zwischen 35 und 39 °C durch ein angepasstes Mittel erwärmt wird, bevor es verwendet wird.

14. Verwendung des Konzentrats nach einem der Ansprüche 1 bis 13 als Nahrungsmittelzusatz.

15. Verwendung des Konzentrats nach einem der Ansprüche 1 bis 13 bei einer Temperatur zwischen 25 und 45 °C, vorteilhafterweise zwischen 35 und 39 °C.

16. Behälter in Form von flexiblen, hermetischen und sterilen Beuteln, die das Konzentrat nach einem der Ansprüche 1 bis 13 enthalten.

17. Mit einem Zusatz versetztes Nahrungsmittel, **dadurch gekennzeichnet, dass** der verwendete Nahrungsmittelszusatz ein flüssiges Konzentrat aus angepassten und lebensfähigen Bakterien nach einem der Ansprüche 1 bis 13 ist.

18. Mit einem Zusatz versetztes Nahrungsmittel nach Anspruch 17, **dadurch gekennzeichnet, dass** es sich um ein Molkereiprodukt und/oder ein Getränk handelt.

19. Verfahren zur Herstellung eines mit einem Zusatz versetzten Nahrungsmittels nach einem der Ansprüche 17 und 18, **dadurch gekennzeichnet, dass** das flüssige Konzentrat aus angepassten und lebensfähigen Bakterien nach einem der Ansprüche 1 bis 13 dem Nahrungsmittel am Ende der Produktionslinie und vorteilhafterweise vor der Verpackung des Nahrungsmittels zugegeben wird.

20. Verfahren zur Herstellung eines mit einem Zusatz versetzten Nahrungsmittels nach Anspruch 19, **dadurch gekennzeichnet, dass** das flüssige Konzentrat aus angepassten und lebensfähigen Bakterien dem Nahrungsmittel in der Linie durch Pumpen zugesetzt wird.
